# EUROPÄISCHE PATENTSCHRIFT

(11) **EP 1 281 383 B1**
(45) Veröffentlichungstag und Bekanntmachung des Hinweises auf die Patenterteilung: **21.09.2005**
(21) Anmeldenummer: 02016298.8
(22) Anmeldetag: 24.07.2002
(51) Int. Cl.: A61K 6/083, A61K 6/093

(54) **Polymerisierbares Dentalmaterial auf der Basis chromophorer Xerogele**
Polymerisable dental material based on chromophoric xerogels
Matériau dentaire basé sur des xerogels chromophoriques

(30) Priorität: 31.07.2001 DE 10137372
(43) Veröffentlichungstag der Anmeldung: 05.02.2003
(73) Patentinhaber: Ivoclar Vivadent AG, 9494 Schaan (LI)
(72) Erfinder: Spange, Stefan, 07768 Orlamünde (DE); Moszner, Norbert, FL-9495 Triesen (LI); Burtscher, Peter, A-6830 Rankweil (AT); Rheinberger, Volker, 9490 Vaduz (LI)
(74) Vertreter: UEXKÜLL & STOLBERG

(56) Entgegenhaltungen:
- EP-A- 0 744 172
- EP-A- 0 948 955
- WO-A-93/00878
- DE-A- 4 002 726
- DE-A- 19 811 900
- DE-A- 19 846 660
- DE-A- 19 950 284
- US-A- 5 583 178
- CLAUDE, CHARLES; GARETZ, BRUCE; OKAMOTO, YOSHIYUKI: "Organically modified silicates that exhibit nonlinear optical properties by the sol-gel technique" POLYMER PREPRINTS (AMERICAN CHEMICAL SOCIETY, DIVISION OF POLYMER CHEMISTRY), Bd. 34, Nr. 1, 1993, Seiten 709-710, XP008010318 NY (USA)

## Beschreibung

Die Erfindung betrifft Dentalmaterialien, die chromophore Xerogele als farbgebende Komponente enthalten.

Dentalmaterialien werden aus ästhetischen Gründen meist mit farbigen Substanzen versetzt, um sie optisch an die natürliche Zahnsubstanz anzupassen. Hierzu werden in der Regel Farbstoffe oder Pigmente verwendet. Bei photopolymerisierbaren Dentalmaterialien tritt das Problem auf, daß sowohl Farbstoffe als auch Pigmente die Durchhärtungstiefe der Materialien nachteilig beeinflussen, d.h. zu einer Verminderung der Durchhärtungstiefe führen. Dieser Effekt tritt verstärkt bei Füllungskompositen auf, bei denen auch die Zugabe von Füllstoffen zu einer zusätzlichen Reduktion der Transparenz des Materials führt. Dementsprechend wird für restaurative Materialien nach dem internationalen Standard ISO 4049 (2000) eine Durchhärtungstiefe von mindestens 1,5 mm gefordert.

Für in Dentalmaterialien eingesetzte Farbmittel bestehen eine Reihe von Grundanforderungen (vgl. L.-A. Linden, Photocuring of polymeric dental materials and plastic composite resins", in: Radiation curing in polymer science and technology, Vol. IV., Hrsg: J.P. Fouassier und J.F. Rabek, Elsevier Appl. Sci., London und New York 1993, 402 ff.), insbesondere Farbstabilität über viele Jahre, Stabilität gegenüber Wärme, beispielsweise bei der Aufnahme von warmer Nahrung, und chemische Stabilität gegenüber Oxidantien oder Reduktionsmitteln. Weiterhin dürfen sich dentale Farbmittel in Fett oder Wasser, Alkohol oder anderen Lösungsmittel nicht lösen, müssen sich gegenüber den anderen Komponenten von Dentalmaterialien inert verhalten und toxikologisch unbedenklich sein. Aus diesen Gründen kommen überwiegend Pigmente zum Einsatz. Die Pigmente lassen sich allgemein in anorganische und organische Pigmente einteilen. Bei den anorganischen Pigmenten handelt es sich häufig um Metalloxide oder Hydroxide, wie z.B. Titandioxid bzw. ZnO als Weißpigment, Eisenoxid (Fe₂0₃) als Rotpigment oder Eisenhydroxid (FeOOH) als Gelbpigment. Die organischen Pigmenten lassen sich u.a. in Azopigmente (z.B. Monoazogelb- und -orangepigmente, Disazopigmente oder α-Naphthol-Pigmente) und Nichtazo- oder polycyclische Pigmente (z.B. Phthalocyanin-, Chinacridon-, Perylen- oder Flavanthron-Pigmente) unterteilen (vgl. W. Herbst und K. Hunger, Industrielle organische Pigmente, VCH, Weinheim 1987, 4 ff.).

Die US 5,583,178 offenbart härtbare Zusammensetzungen, die eine ethylenisch ungesättigte Verbindung, einen Vernetzer mit mehreren SiH-Gruppen, einen Katalysator für die Hydrosilylierung und einen Farbstoff zu Anzeige des Härtungszustands enthalten. Die Zusammensetzungen weisen vor der Härtung eine andere Farbe auf als nach der Härtung.

Claude et al., Polymer Preprints (American Chemical Society, Division of Polymer Chemistry), Bd. 34, Nr. 1, 1993, Seiten 709-710, offenbaren Filme auf der Basis organisch modifizierter Silane, die durch Kokondensation von Trialkoxysilan und N-(3'-(Trialkoxysilyl)propyl)-4-nitroanilin mit N,N-Dimethylacetamid erhalten werden. Die Filme sollen nicht lineare optische Eigenschaften haben.

Der Erfindung liegt die Aufgabe zugrunde, gefärbte Dentalmaterialien mit einer verbesserten Durchhärtungstiefe bei der Photopolymerisation bereitzustellen.

Diese Aufgabe wird durch Dentalmaterialien gelöst, die als farbgebendes Pigment ein chromophores Xerogel der Formel I enthalten:

(Si0₂)ₐ(Si0_{1,5}-Sp-X-CG)(MeₙOₘ)_{b} (1),

in der:
- Me: ein Hauptgruppenmetall der II. bis III. Hauptgruppe oder ein Übergangsmetall der IV. Nebengruppe des Periodensystems der Elemente bedeutet;
- n: 1 oder 2 ist, wobei m gleich 1 oder 2 ist wenn n gleich 1 ist und m gleich 3 ist wenn n gleich 2 ist;
- Sp: ein C₁- bis C₁₀-Alkylenrest oder C₂- bis C₁₀-Oxyalkylenrest mit 1 bis 4 Sauerstoffatomen ist oder entfällt;
- X: eine Verbindungsgruppe, wie CO-O, O-CO, CO-NH, NH-CO, O-CO- NH, NH-CO-O, NR, O, oder S ist oder entfällt, wobei R Wasserstoff oder ein C₁- bis C₆-Alkyl-Rest ist;
- CG: eine 4-Nitrophenyl-Gruppe, aromatische Azogruppe, Di- oder Triarylmethangruppe, Xanthengruppe oder Anthrachinongruppe ist;
- a: eine ganze Zahl von 0 bis 20 ist und
- b: eine ganze Zahl von 0 bis 5 ist.
- X und Sp: entfallen vorzugsweise nicht gleichzeitig.

Bevorzugte Bedeutungen für die angegebenen Variablen sind:
- Me: Ti, Zr und/oder Al;
- Sp: ein C₁- bis C₄-Alkylenrest, vorzugsweise ein Propylenrest;
- X: CO-NH, NH-CO, O-CO-NH, NH-CO-O oder NR;
- R: Wasserstoff oder ein C₁- bis C₄-Alkyl-Rest, vorzugsweise Methyl;
- a: 0, 1, 2, 3, 4 oder 5; und/oder
- b: 0, 1, 2, oder 3.

Unter aromatischen Azogruppen werden Gruppen mit der allgemeinen Struktur R¹-N=N-R² verstanden, wobei R¹ und R² aromatische Reste darstellen.

Die bevorzugten Bedeutungen können unabhängig voneinander gewählt werden. Besonders bevorzugt sind naturgemäß jedoch solche Xerogele, in denen mehrere oder alle Variablen eine der bevorzugten Bedeutungen haben.

Xerogele sind feste Stoffe, die sich aus Kieselgelen bilden, wenn das flüssige Dispersionsmittel durch Verdampfen, Absaugen oder Abpressen entfernt wird. Es handelt sich hierbei um kolloidale, dichte oder lockere, geformte oder ungeformte Kieselsäure, die eine feine Porenstruktur und dadurch bedingt ein hohes Adsorptionsvermögen aufweist (vgl. Ullmanns Encyklopädie der technischen Chemie, 4. Aufl., Band 21, Verlag Chemie; Weinheim etc. 1982, 458 ff.) Kieselgele sehr variierbarer Struktur sind durch die Sol-Gel-Verarbeitung von Mischungen von Kieselsäureestern, z.B. von einem Tetraalkoxysilan mit einem organisch modifizierten Trialkoxysilan, oder mit anderen Metallalkoxiden, wie z.B. Aluminium-, Titan- oder Zirkoniumalkoxiden, zugänglich (vgl. C.J. Brinker und G. W. Scherer, Sol-Gel Science, Academic Press, Bostin etc. 1990, 2 ff.). Bei der hydrolytischen Kondensation solcher Mischungen in Lösung bildet sich primär eine Sol-Phase die in ein Gel übergeht, das aus einen anorganischen Heteropolysiloxan-Netzwerk und dem darin physikalisch eingebundenen Lösungsmittel besteht. Durch Abdampfen des Lösungsmittels erhält man schließlich das entsprechend zusammengesetzte trockene Xerogel. Erfindungsgemäß werden unter Xerogelen vorzugsweise Xerogele im engeren Sinne verstanden, d.h. Xerogele, die im Verlauf der Trocknung, bezogen auf das Hydrogel, eine Volumenkontraktion erleiden.

Die erfindungsgemäßen chromophoren Xerogele sind über den Sol-Gel Prozeß durch hydrolytische Co-Kondensation von Tetraalkoxysilanen, z.B. Tetraethoxysilan (TEOS), mit chromophoren Trialkoxysilanen und gegebenenfalls weiteren Metallalkoxiden zugänglich. Unter chromophoren Trialkoxysilan werden solche Silane verstanden, die als organischen Rest einen Chromophor tragen. Es wird ein gefärbtes Xerogel erhalten, das als chromophores Xerogel bezeichnet wird.

Bevorzugte weitere Metallalkoxide sind Zirkonium- und TitanVerbindungen, wie Zr(OC₂H₅)₄, Zr(0C₃H₇)₄, Zr(0C₄H₉)₄, Ti(OC₂H₅)₄, Ti(OC₃H₇)₄ und Ti(0C₄H⁹)₄. Bevorzugte Aluminium-Verbindungen sind Al(OCH₃)₃, Al(OC₂H₅)₃, Al(OC₃H₇)₃ und Al(OC₄H₉)₃. Die Metallalkoxide werden bei der hydrolytischen Kondensation in die entsprechenden Metalloxide MeₙOₘ überführt. Zur Modifizierung der Kieselgele eignen sich besonders TiO₂, (Me = Ti), Zr0₂ (Me = Zr) oder Al₂0₃ (Me = Al).

Chromophore Trialkoxysilane sind beispielsweise durch die chemische Umsetzung von in 3-Stellung funktionalisierten Propyltrialkoxysilanen, z.B. 3-Amino-, 3-Chloro-, 3-Isocyanato- oder 3-Mercaptopropyltriethoxysilan, mit geeignet funktionalisierten chromophoren Verbindungen erhältlich, z. B.: 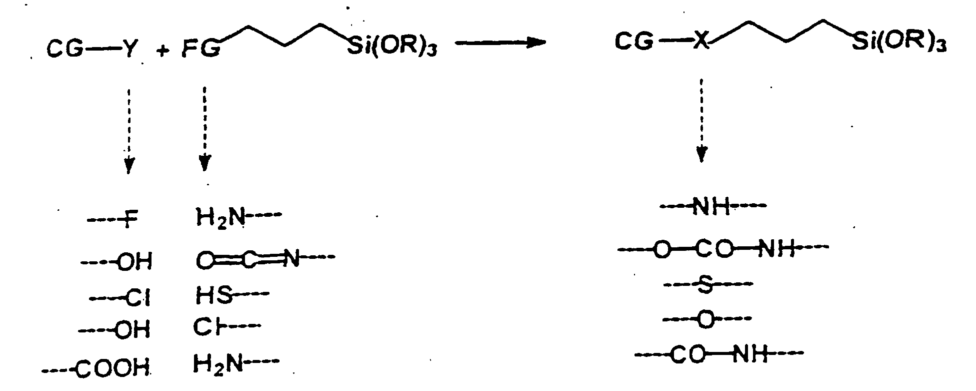 Y und FG stehen jeweils für die funktionellen Gruppen, mit denen die chromophore Verbindung bzw. das Alkyltrialkoxysilan ausgerüstet sein muss, um eine Reaktion in der beschriebenen Weise unter Ausbildung der Gruppe X zu ermöglichen. Y und FG haben vorzugsweise die oben angegebene Bedeutung und werden vorzugsweise auch in den gezeigten Kombinationen miteinander umgesetzt.

Konkretes Beispiel:

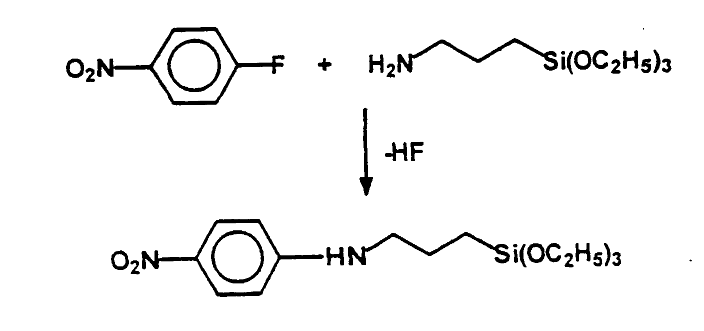

Bei der Herstellung der chromophoren Xerogele durch hydrolytische Kondensation, geht man vorzugsweise so vor, daß man die hydrolysierenden Silane in Gegenwart eines Hydrolyse- und Kondensationskatalysators mit der erforderlichen Menge an Wasser versetzt und die resultierende Mischung rührt. Bevorzugt wird eine Mischung aus TEOS und chromophorem Silan verwendet. Die Silane können entweder als solche oder gelöst in einem geeigneten Lösungsmittel vorliegen. Die Zugabe des Wassers erfolgt bei Raumtemperatur oder unter leichter Kühlung. Nach der Bildung des Gels läßt man den Reaktionsansatz zur vollständigen Umsetzung längere Zeit stehen und trennt dann das verwendete Lösungsmittel durch Erwärmen oder Evakuieren ab. Als Lösungsmittel kommen vor allem aliphatische Alkohole, wie z. B. Ethanol oder i-Propanol, Dialkylketone, wie Aceton oder Methylisobutylketon, Ether, wie z. B. Diethylether oder Tetrahydrofuran (THF), Ester, wie Ethyl- oder Butylacetat, und deren Gemische in Frage. Das erhaltene Xerogels kann zur Abtrennung von nicht reagierten Komponenten nochmals mit einem inerten Lösungsmittel gewaschen und dann bis zur Gewichtskonstanz getrocknet werden. Wird die hydrolytische Kondensation in Gegenwart reaktiver Zr-, Ti- oder Al-Verbindungen durchgeführt, sollte die Wasserzugabe stufenweise bei ca. 0 bis 30 °C erfolgen. Dabei ist es bevorzugt, das Wasser nicht als solches sondern in Form von wasserhaltigen Lösungsmitteln, wie z.B. wässrigem Ethanol, zuzugeben oder durch eine chemische Reaktion freizusetzen, beispielsweise durch eine Veresterung.

Besonders bevorzugt wird die Synthese des chromophoren Silans und die hydrolytische Kondensation in einem Eintopfverfahren durch geführt. Dies ist z.B. dann möglich, wenn bei der Synthese des chromophoren Silans TEOS als Lösungsmittel verwendet werden kann und gleichzeitig Fluorwasserstoff freigesetzt wird, der bei der hydrolytischen Kondensation als Katalysator wirkt. Nach der Herstellung des Silans muß zur Gelbildung nur noch Wasser zugegeben werden.

Bevorzugt sind Xerogele mit einer spezifischen Oberfläche nach BET von 50 bis 800 m²/g, insbesondere 200 bis 600 m²/g. Die mittels Rasterelektronenmikroskopie (REM) bestimmte Primärpartikelgröße der Xerogele liegt vorzugsweise im Bereich von 0,1 bis 20 µm, insbesondere 0,1 bis 5 um. Bei der Partikelgröße handelt es sich um einen zahlengemittelten Wert. Die mittels BJH-Methode (Barnett E.T. et al.; J.Amer.Soc. 73 (1951) 373) bestimmte Porengröße liegt vorzugsweise im Bereich von 1 bis 15 nm, insbesondere 1 bis 10 nm. Der Restgehalt an Wasser oder Lösungsmittel sollte unter 2,0 Gew.-%, vorzugsweise unter 1,0 Gew.-% liegen.

Unter Chromophor, chromophorer Gruppe oder chromophorem System werden die farbgebenden Reste verstanden, die eine Lichtabsorption im Sichtbaren hervorrufen, und nicht nur einzelne Atomgruppierungen mit n-Elektronen (C=C, C=O, C=S, N=O, C=N), deren Absorptionsspektrum oft im Ultravioletten liegt (M. Klessinger, Chemie in unserer Zeit, 12 (1978), 1-10).

Als Chromophor werden vorzugsweise einfache Gruppen eingesetzt, die farbgebend sind, z.B. 4-Nitrophenyl-Derivate, oder auch Gruppen, die sich aus den bekannten Farbstoffklassen, wie z. B. Azofarbstoffen, Di- und Triarylmethanfarbstoffen, Xanthenfarbstoffen oder Anthrachinonfarbstoffen, ableiten und die sich auf die radikalische Polymerisation nicht nachteilig auswirken.

Die Anbindung des Chromophors an das Heteropolysiloxangerüst erfolgt vorzugsweise über einen Spacer Sp, wobei die Propylen-Gruppen als Spacer besonders bevorzugt ist. In 3-Stellung funktionalisierte Propyltrialkoxysilane, die als Ausgangskomponente bei dem Spacer dienen, sind kommerziell erhältlich.

Als Verbindungsgruppe X sind CO-NH, NH-CO, O-CO-NH, NH-CO-O und NR (mit R = H oder CH₃) besonders geeignet. Die Verbindungsgruppe dient in erster Linie zur Anbindung der chromophoren Gruppe an den Spacer Sp und damit auch an die hydrolytisch kondensierbare Trialkoxysilylgruppe.

Die chromophoren Xerogele zeichnen sich durch eine hohe Farbstabilität über viele Jahre, Stabilität gegenüber Wärme, wichtig z.B. bei der Aufnahme von warmer Nahrung, und chemische Stabilität gegenüber Oxidantien oder Reduktionsmitteln aus. Weiterhin sind sie in Fett, Wasser, Alkohol oder anderen Lösungsmitteln nicht löslich und verhalten sich gegenüber den übrigen Komponenten von Dentalmaterialien inert. Außerdem sind sie toxikologisch unbedenklich.

Zur Herstellung der erfindungsgemäßen Dentalmaterialien wird das chromophore Xerogel mit einem polymerisierbaren Bindemittel, vorzugsweise einem organischen polymerisierbaren Bindemittel gemischt. Hierbei wird in der Regel eine Dispersion des Xerogels in dem Bindemittel erhalten. Anschließend wird die Mischung mit einem Initiator für die radikalische Polymerisation, vorzugsweise einem Photoinitiator, und gegebenenfalls mit weiteren Additiven und Füllstoffen versetzt.

Zur Einfärbung der Dentalmaterialien sind bereits geringe Mengen an chromophoren Xerogelen ausreichend, so daß diese vorzugsweise in einer Menge von 0,0001 bis 1,0 Gew.-%, besonders bevorzugt 0,001 bis 0,1 Gew.-% bezogen auf die Gesamtmasse des Dentalmaterials eingesetzt werden.

Als radikalisch polymerisierbare Bindemittel eignen sich besonders polymerisierbare Monomere, wie Mono(meth)acrylate, z.B. Methyl-, Ethyl-, Butyl-, Benzyl-, Furfuryl- oder Phenyl-(meth)acrylat, sowie die als Vernetzermonomere bekannten mehrfunktionellen Acrylate und Methacrylate, beispielsweise Dimethacrylate, wie Urethandimethacrylate, Bisphenol-A-di(meth)acrylat, Bis-GMA (ein Additionsprodukt aus Methacrylsäure und Bisphenol-A-diglycidylether), UDMA (ein Additionsprodukt aus 2-Hydroxyethylmethacrylat und 2,2,4-Trimethylhexamethylendiisocyanat), Di-, Tri- oder Tetraethylenglycoldi(meth)acrylat, Decandioldi-(meth)acrylat, Trimethylolpropantri(meth)acrylat, Pentaerythrittetra(meth)acrylat, sowie Butandioldi(meth)acrylat, 1,10-Decandioldi(meth)acrylat oder 1,12-Dodecandioldi(meth)acrylat sowie Mischungen dieser Monomere.

Der Bindemittelgehalt liegt vorzugsweise im Bereich von 10 bis 90 Gew.-%, besonders bevorzugt 10 bis 80 Gew.-%, bezogen auf die Gesamtmasse des Dentalmaterials.

Zur Initiierung der radikalischen Photopolymerisation werden vorzugsweise Benzophenon, Benzoin sowie deren Derivate oder α-Diketone oder deren Derivate, wie 9,10-Phenanthrenchinon, Diacetyl oder 4,4-Dichlorbenzil eingesetzt. Bevorzugt werden Campherchinon und 2,2-Methoxy-2-phenyl-acetophenon und besonders bevorzugt α-Diketone, wie Campherchinon, in Kombination mit Aminen als Reduktionsmittel, vorzugsweise tertiären Aminen, wie z.B. 4-(N,N-Dimethylamino)-benzoesäureester, N,N-Dimethylaminoethylmethacrylat, N,N-Dimethyl-sym.-xylidin oder Triethanolamin, verwendet. Darüber hinaus sind auch Acylphosphine, wie z.B. 2,4,6-Trimethylbenzoyldiphenyl- oder Bis(2,6-dichlorbenzoyl)-4-N-propylphenylphosphinoxid besonders geeignet.

Initiatoren werde vorzugsweise in einer Menge von 0,1 bis 5,0 Gew.-%, besonders bevorzugt 0,2 bis 2,0 Gew.-% eingesetzt, jeweils bezogen auf die Gesamtmasse des Dentalmaterials.

Weiterhin können die erfindungsgemäßen Dentalmaterialien zur Verbesserung der mechanischen Eigenschaften mit organischen oder anorganischen Partikeln oder Fasern gefüllt werden. Bevorzugte anorganische partikuläre Füllstoffe sind amorphe kugelförmige Materialien auf der Basis von Oxiden, wie Zr0₂ und Ti0₂, oder Mischoxiden aus Si0₂, ZrO₂ und/oder Ti0₂, Metalloxide mit einer Primärpartikelgröße von ca. 40 bis 300 nm, Splitterpolymerisate mit einer Partikelgröße von 10-100 µm (vgl. R. Janda, Kunststoffverbundsysteme, VCH Verlagsgesellschaft, Weinheim, 1990, Seite 225 ff.), nanopartikuläre oder mikrofeine Füllstoffe, wie pyrogene Kieselsäure oder Fällungskieselsäure, sowie Makro- oder Minifüllstoffe, wie Quarz-, Glaskeramik- oder Glaspulver mit einer durchschnittlichen Teilchengröße von 0,01 bis 5-µm, sowie röntgenopake Füllstoffe, wie Ytterbiumtrifluorid und gemahlene röntgenopake Gläser mit einer mittleren Partikelgröße zwischen ca. 0,5 und 5 µm. Darüber hinaus können auch Glasfasern, Polyamid- oder Kohlenstoff-Fasern eingesetzt werden.

Der Füllstoffgehalt richtet sich nach der gewünschten Verwendung des Dentalmaterials. Zemente enthalten vorzugsweise 5 bis 60 Gew.-%, insbesondere 20 bis 60 Gew.-%, Verblendmaterialien vorzugsweise 50 bis 90 Gew.-%, insbesondere 60 bis 80 Gew.-% und Füllungskomposite vorzugsweise 50 bis 90 Gew.-%, insbesondere 75 bis 85 Gew.-% Füllstoff, jeweils bezogen auf die Gesamtmasse des Dentalmaterials.

Gegebenenfalls können die erfindungsgemäß eingesetzten Zusammensetzungen weitere Additive enthalten, wie z.B. Stabilisatoren, Aromastoffe, mikrobizide Wirkstoffe, optische Aufheller, Weichmacher oder UV-Absorber, Inhibitoren zur Verhinderung einer vorzeitigen Polymerisation und Photostabilisatoren zum Schutz gegen photooxidativen Abbau. Diese Additive werden vorzugsweise in geringen Mengen von insgesamt 0,1 bis 3,0, insbesondere 0,1 bis 1,0 Gew.-% eingesetzt, bezogen auf die Gesamtmasse des Dentalmaterials.

Die erfindungsgemäßen Dentalmaterialien zeigen bei der Polymerisation im Vergleich zu Materialien, mit herkömmlichen Pigmenten überraschenderweise eine größere Durchhärtungstiefe. Zudem kann der Gehalt an chromophoren Xerogel im Vergleich zu herkömmlichen Pigmenten deutlich reduziert werden kann, ohne daß eine Minderung der Farbintensität auftritt.

Bevorzugt sind Dentalmaterialien, die
(a) 0,0001 bis 1,0 Gew.-%, insbesondere 0,001 bis 0,1 Gew.-% chromophores Xerogel der allgemeinen Formel (I),
(b) 10 bis 98 Gew.-%, insbesondere 10 bis 80 Gew.-% polymerisierbares Bindemittel,
(c) 0,1 bis 5,0 Gew.-%, insbesondere 0,2 bis 2,0 Gew.-% Polymerisationsinitiator und
(d) 0 bis 90 Gew.-%, insbesondere 0 bis 80 Gew.-% Füllstoffe,
enthalten, jeweils bezogen auf die Gesamtmasse des Dentalmaterials.

Die Dentalmaterialien eignen sich besonders als Zemente, Verblendmaterialien und insbesondere Füllungskomposite.

Ein besonders bevorzugter Zement enthält:
(a) 0,0001 bis 1,0 Gew.-%, insbesondere 0,001 bis 0,1 Gew.-% chromophores Xerogel der allgemeinen Formel (I),
(b) 10 bis 90 Gew.-%, insbesondere 20 bis 50 Gew.-% polymerisierbares Bindemittel,
(c) 0,1 bis 5,0 Gew.-%, insbesondere 0,2 bis 2,0 Gew.-% Polymerisationsinitiator und
(d) 5 bis 60 Gew.-%, insbesondere 20 bis 60 Gew. % Füllstoffe, jeweils bezogen auf die Gesamtmasse des Zements.

Ein besonders bevorzugtes Verblendmaterial enthält:
(a) 0,0001 bis 1,0 Gew.-%, insbesondere 0,001 bis 0,1 Gew.-% chromophores Xerogel der allgemeinen Formel (I),
(b) 10 bis 50 Gew.-%, insbesondere 10 bis 30 Gew.-% polymerisierbares Bindemittel,
(c) 0,1 bis 5,0 Gew.-%, insbesondere 0,2 bis 2,0 Gew.-% Polymerisationsinitiator und
(d) 50 bis 90 Gew.-%, insbesondere 60 bis 80 Gew. % Füllstoffe, jeweils bezogen auf die Gesamtmasse des Verblendmaterials.

Ein besonders bevorzugtes Füllungskomposit enthält:
(a) 0,0001 bis 1,0 Gew.-%, insbesondere 0,001 bis 0,1 Gew.-% chromophores Xerogel der allgemeinen Formel (I),
(b) 10 bis 50 Gew.-%, insbesondere 15 bis 25 Gew.-% polymerisierbares Bindemittel,
(c) 0,1 bis 5,0 Gew.-%, insbesondere 0,2 bis 2,0 Gew.-% Polymerisationsinitiator und
(d) 50 bis 90 Gew.-%, insbesondere 75 bis 85 Gew. % Füllstoffe, jeweils bezogen auf die Gesamtmasse des Komposits.

Die Erfindung wird im Folgenden anhand von Beispielen näher erläutert.

### Beispiele

### Beispiel 1: Synthese eines chromophoren Xerogels

3,527 g 4-Fluornitrobenzol (0,025 mol), 25,88 ml Tetraethoxysilan (26,041 g, 0,116 mol) und 11,03 ml 3-Aminopropyltrimethoxysilan (11,2056 g, 0,0625 mol) wurden in einem Dreihalskolben durch Rühren vermischt. Es bildete sich eine hellgelbe, leicht trübe Lösung. Diese wurde bei 130 °C für 5 Stunden gekocht. Dabei wurde das Reaktionsgemisch klar und dunkler. Der Ansatz wurde langsam abgekühlt und über Nacht stehen gelassen. Es wurden dann 50 ml Ethanol zugefügt, woraufhin eine leichte Trübung feststellbar war. Dann wurden 3 ml Wasser zugetropft. Dabei trat eine Erwärmung ein und das Gemisch wurde dicker und trüber. Es wurden weitere 100 ml Wasser zugegeben und die Suspension dann für 2 Stunden gerührt und anschließend abfiltriert. Es wurde mit Wasser und Ethanol gewaschen und im Exsikkator über Calciumchlorid (Vakuum) bis zur Gewichtskonstanz getrocknet. Es ergab sich eine Gesamtausbeute von ca. 90 %.

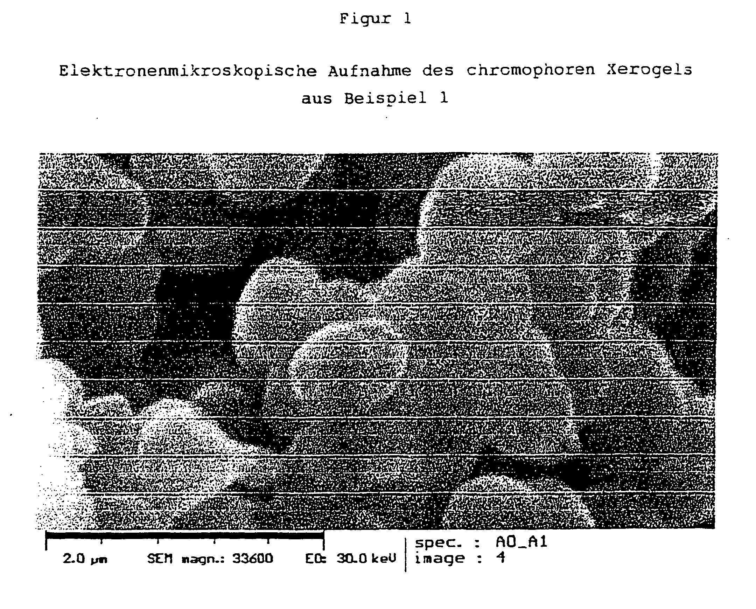

Das chromophore Xerogel wurde mittels Elektronenmikroskopie und ¹³C-MAS-CP-Festkörper-NMR untersucht. Eine elektronenmikroskopische Aufnahme des Produkts ist in Figur 1 gezeigt, die NMR-Untersuchung ergab das folgende Ergebnis:

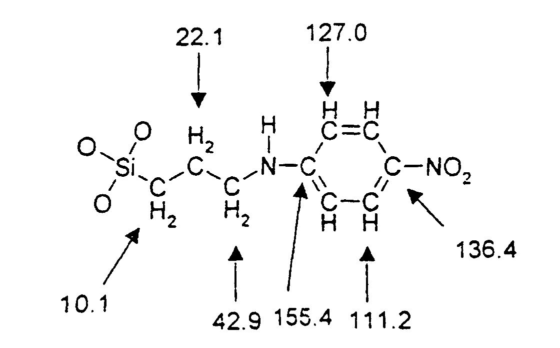

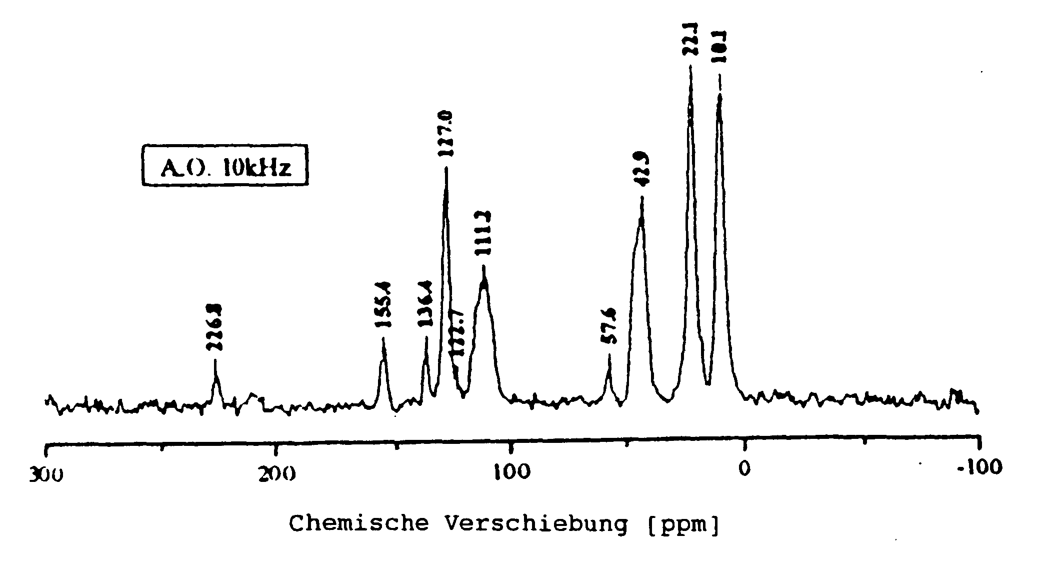

### Beispiel 2: Herstellung eines gelb eingefärbten dentalen Komposites auf der Basis eines chromophoren Xerogels

Um Komposite zahnfarben einzufärben, werden vor allem Gelb-Pigmente auf Eisenoxidbasis (Lichtgelb 8G) verwendet. Dementsprechend wurde ein so eingefärbtes Komposit als Vergleichsbeispiel ausgewählt. Dabei wurde in einem Vorversuch ermittelt, welche Konzentration von chromophorem Xerogel notwendig ist, um die gleiche Farbwirkung wie mit Lichtgelb 8G zu erhalten.

| **Zusammensetzung der Kompositpasten (Angaben in Gew-%) und deren Durchhärtungstiefe bei der Photopolymerisation:** | | |
|---|---|---|
| | **Vergleichspaste** | **Xerogel-Paste** |
| Monomer | 21,8 | 21,8 |
| Füller | 77,9 | 78,19 |
| Lichtgelb 8G | 0,3 | 0 |
| Xerogel aus Bsp. 1 | 0 | 0,01 |
| Durchhärtungstiefe (mm) | 3,9 | 4,9 |

Bei der Methode zur Bestimmung der Durchhärtungstiefe wurde gemäß ISO-4049 verfahren. Eine zylindrische Öffnung in einer Stahlform (h=6 mm, d=4 mm) wurde mit der Kompositpaste gefüllt und mit einem Polymerisationsgerät 40 s polymerisiert. Im Anschluß daran wurde das Komposit aus der Metallform entfernt und der nichtpolymerisierte Anteil mit einem Kunststoffspatel vom ausgehärteten Teil abgekratzt. Dann wurde mit einer Schieblehre die Dicke des ausgehärteten Komposites bestimmt. Für die Untersuchungen wurde die Polymerisationslampe Astralis 5 (Vivadent) verwendet.

Die Versuche zeigten, daß mit einer Konzentration von 0,01 % chromophorem Xerogel der gleiche Farbeffekt erreicht wird, wie mit 0,3 % Lichtgelb 8G. Es wurde eine deutlich höhere Durchhärtungstiefe (+ 25 %) erzielt.

### Beispiel 3: Herstellung eines dunkel eingefärbten dentalen Komposites auf der Basis eines chromophoren Xerogels

In zahnfarbenen Kompositen werden neben Gelb-Pigmenten auch rote, braune und schwarze Pigmente eingesetzt, um eine optimale Zahnfarbe zu erhalten. Es wurde ein Komposit in der Vita-Farbe A3.5 hergestellt, einer relativ dunklen Farbe, wobei wiederum die Gelb-Pigmente Lichtgelb 8 G und das chromophore Xerogel verglichen wurden.

| **Zusammensetzung der Kompositpasten (Angaben in Gew.-%) und deren Durchhärtungstiefe bei der Photopolymerisation** | | |
|---|---|---|
| | **Vergleichspaste** | **Xerogel-Paste** |
| Monomer und Füller | 99,9347 | 99,9582 |
| Braun-Pigment | 0,0301 | 0,0301 |
| Rot-Pigment | 0,0103 | 0,0103 |
| Schwarz-Pigment | 0,0004 | 0,0004 |
| Lichtgelb 8 G | 0,0245 | 0 |
| Xerogel aus Bsp. 1 | 0 | 0,0010 |
| Durchhärtungstiefe (mm) | 3,6 | 4,0 |

Auch bei diesem sehr dunklen Komposit zeigte sich der positive Effekt der chromophoren Xerogele. Bei gleicher Farbe wird eine Verbesserung in der Durchhärtungstiefe von 0,4 mm erreicht, was bei dunklen Farben einen signifikanten Unterschied darstellt.

## Patentansprüche

1. Dentalmaterial auf der Basis polymerisierbarer Bindemittel, **dadurch gekennzeichnet, daß** es mindestens ein chromophores Xerogel der Formel I enthält:
(Si0₂)ₐ(Si0_{1,5}-Sp-X-CG)(MeₙOₘ)_{b} (1),
in der:
Me ein Hauptgruppenmetall der II. bis III. Hauptgruppe oder ein Übergangsmetall der IV. Nebengruppe des Periodensystems der Elemente bedeutet;
n 1 oder 2 ist, wobei m gleich 1 oder 2 ist wenn n gleich 1 ist und m gleich 3 ist wenn n gleich 2 ist;
Sp ein C₁- bis C₁₀-Alkylenrest oder C₂- bis C₁₀-Oxyalkylenrest mit 1 bis 4 Sauerstoffatomen ist oder entfällt;
X eine Verbindungsgruppe, wie CO-O, O-CO, CO-NH, NH-CO, O-CO-NH, NH-CO-O, NR, O, oder S ist oder entfällt, wobei R Wasserstoff oder ein C₁- bis C₆-Alkyl-Rest ist;
CG eine 4-Nitrophenyl-Gruppe, aromatische Azogruppe, Di- oder Triarylmethängruppe, Xanthengruppe oder Anthrachinongruppe ist;
a eine ganze Zahl von 0 bis 20 ist und
b eine ganze Zahl von 0 bis 5 ist.

2. Dentalmaterial nach Anspruch 1, **dadurch gekennzeichnet, daß** Me Ti, Zr und/oder Al ist;
Sp ein C₁- bis C₄-Alkylenrest, vorzugsweise ein Propylenrest ist;
X CO-NH, NH-CO, O-CO-NH, NH-CO-O oder NR ist; wobei
R Wasserstoff oder ein C₁- bis C₄-Alkyl-Rest, vorzugsweise Methyl ist;
a 0, 1, 2, 3, 4 oder 5 ist und
b 0, 1, 2, oder 3 ist.

3. Dentalmaterial nach Anspruch 1 oder 2, **dadurch gekennzeichnet, daß** es einen Initiator für die radikalische Photopolymerisation enthält.

4. Dentalmaterial nach Anspruch 3, **dadurch gekennzeichnet, daß** es als .Initiator ein Benzophenon, Benzoin, α-Diketon, wie 9,10-Phenanthrenchinon, Diacetyl, 4,4-Dichlorbenzil, Campherchinon, 2,2-Methoxy-2-phenyl-acetophenon, Acylphosphin, wie 2,4,6-Trimethylbenzoyldiphenyl- oder Bis(2,6-dichlorbenzoyl)-4-N-propylphenylphosphinoxid, oder ein Derivat der genannten Verbindungen enthält.

5. Dentalmaterial nach Anspruch 3 oder 4, **dadurch gekennzeichnet, daß** es zusätzlich ein Amin als Reduktionsmittel enthält, vorzugsweise 4-(N,N-Dimethylamino)-benzoesäureester, N,N-Dimethylaminoethylmethacrylat, N,N-Dimethyl-sym.-xylidin und/oder Triethanolamin.

6. Dentalmaterial nach einem der Ansprüche 1 bis 5, **dadurch gekennzeichnet, daß** es als polymerisierbares Bindemittel Mono(meth)acrylat, z.B. Methyl-, Ethyl-, Butyl-, Benzyl-, Furfuryl- oder Phenyl(meth)acrylat, mehrfunktionelles (Meth)acrylat, wie z.B. Bisphenol-A-di(meth)acrylat, Bis-GMA (ein Additionsprodukt aus Methacrylsäure und Bisphenol-A-diglycidylether), UDMA (ein Additionsprodukt aus 2-Hydroxyethylmethacrylat und 2,2,4-Trimethylhexamethylendiisocyanat), Di-, Tri- oder Tetraethylenglycoldi(meth)acrylat, Decandioldi(meth)acrylat,Trimethylolpropantri(meth)acrylat, Pentaerythrittetra(meth)acrylat, Butandioldi(meth)acrylat, 1,10-Decandioldi(meth)acrylat oder 1,12-Dodecandiol-di(meth)acrylat oder eine Mischung dieser Verbindungen enthält.

7. Dentalmaterial nach einem der Ansprüche 1 bis 6, **dadurch gekennzeichnet, daß** es anorganischen oder organischen, teilchenförmigen oder faserförmigen Füllstoff enthält.

8. Dentalmaterial nach einem der Ansprüche 1 bis 7, **dadurch gekennzeichnet, daß** es
(a) 0,0001 bis 1,0 Gew.-%, vorzugsweise 0,001 bis 0,1 Gew.-%, chromophores Xerogel der Formel (I);
(b) 10 bis 98 Gew.-%, vorzugsweise 10 bis 80 Gew.-% polymerisierbares Bindemittel; und
(c) 0,1 bis 5,0 Gew.-%, vorzugsweise 0,2 bis 2,0 Gew.-% Polymerisationsinitiator
enthält, jeweils bezogen auf die Gesamtmasse des Dentalmaterials.

9. Dentalmaterial nach Anspruch 8, **dadurch gekennzeichnet, daß** es als weitere Komponente (d) 0 bis 90 Gew.-%, vorzugsweise 0 bis 80 Gew. % Füllstoff enthält, bezogen auf die Gesamtmasse des Dentalmaterials.

10. Verwendung eines chromophoren Xerogels der Formel I:
(Si0₂)ₐ(Si0_{1,5}-Sp-X-CG)(MeₙOₘ)_{b} (1),
in der:
Me ein Hauptgruppenmetall der II. bis III. Hauptgruppe oder ein Übergangsmetall der IV. Nebengruppe des Periodensystems der Elemente bedeutet;
n 1 oder 2 ist, wobei m gleich 1 oder 2 ist wenn n gleich 1 ist und m gleich 3 ist wenn n gleich 2 ist;
Sp ein C₁- bis C₁₀-Alkylenrest oder C₂- bis C₁₀-Oxyalkylenrest mit 1 bis 4 Sauerstoffatomen ist oder entfällt;
X eine Verbindungsgruppe, wie CO-O, O-CO, CO-NH, NH-CO, O-CO-NH, NH-CO-O, NR, O, oder S ist oder entfällt, wobei R Wasserstoff oder ein C₁- bis C₆-Alkyl-Rest ist;
CG eine 4-Nitrophenyl-Gruppe, aromatische Azogruppe, Di- oder Triarylmethängruppe, Xanthengruppe oder Anthrachinongruppe ist;
a eine ganze Zahl von 0 bis 20 ist und
b eine ganze Zahl von 0 bis 5 ist
zur Herstellung eines Dentalmaterials.

11. Verwendung nach Anspruch 10 zur Herstellung eines photohärtbaren Dentalmaterials.

12. Verwendung nach Anspruch 10 oder 11 zur Herstellung von Zementen, Verblendmaterialien oder Füllungskompositen.

## Claims

1. Dental material based on polymerisable binders, **characterised in that** it comprises at least one chromophoric xerogel of Formula 1:
(SiO₂)ₐ(SiO_{1.5}-Sp-X-CG)(MeₙOₘ)_{b} (1),
in which:
Me means a main-group metal of the II^{nd} to III^{rd} main groups or a transition metal of the IV^{th} sub-group of the periodic table of the elements;
n is 1 or 2, m being equal to 1 or 2 if n is equal to 1 and m equals 3 if n is equal to 2;
Sp is a C₁ to C₁₀ alkylene radical or C₂ to C₁₀ oxyalkylene radical with 1 to 4 oxygen atoms or is absent;
X is a linking group, such as CO-O, O-CO, CO-NH, NH-CO, O-CO-NH, NH-CO-O, NR, O, or S or is absent, R being hydrogen or a C₁ to C₆ alkyl radical;
CG is a 4-nitrophenyl group, aromatic azo group, di-or triarylmethane group, xanthene group or anthraquinone group;
a is a whole number from 0 to 20 and
b is a whole number from 0 to 5.

2. Dental material according to Claim 1, **characterised in that**
Me is Ti, Zr and/or Al;
Sp is a C₁ to C₄ alkyl radical, preferably a propyl radical;
X is CO-NH, NH-CO, O-CO-NH, NH-CO-O or NR; wherein
R is hydrogen or a C₁ to C₄ alkyl radical, preferably methyl;
a is 0, 1, 2, 3, 4 or is 5 and
b is 0, 1, 2, or is 3.

3. Dental material according to Claim 1 or 2, **characterised in that** it comprises an initiator for radical photopolymerisation.

4. Dental material according to Claim 3, **characterised in that** it comprises as the initiator a benzophenone, benzoin, α-diketone, such as 9,10-phenanthrenequinone, diacetyl, 4,4-dichlorobenzil, camphorquinone, 2,2-methoxy-2-phenyl-acetophenone, acylphosphine, such as 2,4,6-trimethylbenzoyldiphenyl- or bis(2,6-dichlorobenzoyl)-4-n-propylphenylphosphine oxide, or a derivative of the cited compounds.

5. Dental material according to Claim 3 or 4, **characterised in that** it further comprises an amine as the reducing agent, preferably 4-(N,N-dimethylamino)-benzoic acid ester, N,N-dimethylaminoethyl methacrylate, N,N-dimethyl-*sym*.xylidine and/or triethanolamine.

6. Dental material according to one of Claims 1 to 5, **characterised in that** it comprises as the polymerisable binder mono(meth)acrylate, e.g. methyl-, ethyl-, butyl-, benzyl-, furfuryl- or phenyl (meth)acrylate, multifunctional (meth)acrylate, such as e.g. bisphenol-A-di(meth)acrylate, bis-GMA (an addition product of methacrylic acid and bisphenol-A-diglycidyl ether), UDMA (an addition product of 2-hydroxyethyl methacrylate and 2,2,4-trimethylhexamethylene diisocyanate), di-, tri- or tetraethyleneglycol di(meth)acrylate, decanediol di(meth)acrylate, trimethylolpropane tri(meth)acrylate, pentaerythreitol tetra(meth)acrylate, butanediol di(meth)acrylate, 1,10-decanediol di(meth)acrylate or 1,12-dodecanediol di(meth)acrylate or a mixture of these compounds.

7. Dental material according to one of Claims 1 to 6, **characterised in that** it comprises inorganic or organic particulate or fibrous filler.

8. Dental material according to one of Claims 1 to 7, **characterised in that** it comprises
a) 0.0001 to 1.0 wt.%, preferably 0.001 to 0.1 wt.% chromophoric xerogel of the Formula (1);
b) 10 to 98 wt.%, preferably 10 to 80 wt.% polymerisable binder; and
c) 0.1 to 5.0 wt.%, preferably 0.2 to 2.0 wt.% polymerisation initiator, each based on the total weight of the dental material.

9. Dental material according to Claim 8, **characterised in that** it comprises as additional component (d) 0 to 90 wt.%, preferably 0 to 80 wt.% filler, based on the total weight of the dental material.

10. Use of a chromophoric xerogel of Formula (1):
(SiO₂)ₐ(SiO_{1.5}-Sp-X-CG)(MeₙOₘ)_{b} (1),
in which:
Me means a main-group metal of the II^{nd} to III^{rd} main groups or a transition metal of the IV^{th} sub-group of the periodic table of the elements;
n is 1 or 2, m being equal to 1 or 2 if n is equal to 1 and m equals 3 if n is equal to 2;
Sp is a C₁-to C₁₀ alkylene radical or C₂ to C₁₀ oxyalkylene radical with 1 to 4 oxygen atoms or is absent;
X is a linking group, such as CO-O, O-CO, CO-NH, NH-CO, O-CO- NH, NH-CO-O, NR, O, or S or is absent, wherein
R is hydrogen or a C₁ to C₆ alkyl radical;
CG is a 4-nitrophenyl group, aromatic azo group, di-or triarylmethane group, xanthene group or anthraquinone group;
a is a whole number from 0 to 20 and
b is a whole number from 0 to 5
for the manufacture of a dental material.

11. Use according to Claim 10 for the manufacture of a photocurable dental material.

12. Use according to Claim 10 or 11 for the manufacture of cements, facing materials or filling composites.

## Revendications

1. Matériau dentaire fabriqué à base de liants polymérisables, **caractérisé en ce que** ledit matériau comprend au moins un xérogel chromophorique de la formule I :
(Si0₂)ₐ(Si0 _{1,5}-Sp-X-CG) (MeₙOₘ)b
dans laquelle :
Me est un métal de groupe principal du IIème au IIIème groupe principal ou un métal de transition du IVème groupe auxiliaire du système périodique des éléments ;
n est 1 ou 2, m étant égal à 1 ou 2, lorsque n est égal à 1 et m étant égal à 3, lorsque n est égal à 2 ;
Sp est un reste alkylène en C₁ à C₁₀ ou un reste oxyalkylène en C₂ à C₁₀ avec 1 à 4 atomes d'oxygène, ou absent;
X est un groupe de liaison tel que CO-O, O-CO, CO-NH, NH-CO, O-CO-NH, NH-CO-O, NR, O, ou S, ou absent, R étant hydrogène ou un reste alkyle en C₁ à C₆;
CG est un groupe 4-nitrophényle, un groupe azoïque aromatique, un groupe diarylméthane ou triarylméthane, un groupe xanthène ou un groupe anthrachinone ;
a est un nombre entier de 0 à 20, et
b est un nombre entier de 0 à 5.

2. Matériau dentaire selon la revendication 1, **caractérisé en ce que**
Me est du Ti, Zr et/ou Al ;
Sp est un reste alkylène en C₁ à C₄, préférentiellement un reste de propylène ;
X est CO-NH, NH-CO, O-CO-NH, NH-CO-O ou NR ;
R étant hydrogène ou un reste alkyle en C₁ à C₄, préférentiellement méthyle ;
a est 0, 1, 2, 3, 4 ou 5 ; et
b étant 0, 1, 2 ou 3.

3. Matériau dentaire selon la revendication 1 ou 2, **caractérisé en ce qu'**il contient un initiateur pour la photopolymérisation radicalaire.

4. Matériau dentaire selon la revendication 3, **caractérisé en ce qu'**il contient comme initiateur une benzophénone, benzoïne, α-dicétone telles que 9,10-phénanthrènechinone, diacétyle, 4,4-dichlorbenzyle, campherchinone, 2,2-méthoxy-2-phényl-acétophénone, acylphosphine telle que 2,4,6-triméthylbenzoyldiphényle- ou Bis (2,6-dichlorbenzoyl)-4-N-propylphénylphosphinoxide ou un dérivé des composés mentionnés.

5. Matériau dentaire selon la revendication 3 ou 4, **caractérisé en ce qu'**il contient en plus une amine comme agent réducteur, préférentiellement un ester d'acide le 4-(N,N-diméthylamino) benzoïque, N,N-diméthylaminoéthylmethacrylate, N,N-diméthyl-sym.-xylidine et/ou triéthanolamine.

6. Matériau dentaire selon l'une quelconque des revendications 1 à 5, **caractérisé en ce qu'**il contient comme liant polymérisable du mono(méth)acrylate, par ex. le méthyl(méth)acrylate, l'éthyl(méth)acrylate, le butyl(méth)acrylate, le benzyl(méth)acrylate, le furfuryl(méth)acrylate ou le phényl(méth)acrylate, de même que du (méth)acrylate multifonctionnel tel que le bisphénol-A-di(méth)acrylate, bis-GMA (un produit d'addition dérivé de l'acide méthacrylique et du bisphénol-A-diglycidyléther), UDMA (un produit d'addition dérivé du 2-hydroxyéthylméthacrylate et du 2,2,4-triméthylhexaméthylène diisocyanate), le di-, tri- ou tétraéthylène glycoldi(méth)acrylate, le décanedioldi(méth)acrylate, le triméthylolpropanetri(méth)acrylate, le pentaérythritetetra(méth)acrylate, de même que le butandioldi(méth)acrylate, 1,10-decanedioldi(méth)acrylate ou le 1,12-dodecanedioldi(méth)acrylate, ou un mélange de ces composés.

7. Matériau dentaire selon l'une quelconque des revendications 1 à 6, **caractérisé en qu'**il contient de la matière de charge inorganique ou organique, en forme de particules ou en forme de fibres.

8. Matériau dentaire selon l'une quelconque des revendications 1 à 7, **caractérisé en qu'**il contient :
(a) 0,0001 à 1,0 % en poids, préférentiellement 0,001 à 0,1 % en poids de xérogel chromophorique de la formule générale (I),
(b) 10 à 98 % en poids, préférentiellement 10 à 80 % en poids de liant polymérisable, et
(c) 0,1 à 5,0 % en poids, préférentiellement 0,2 à 2,0 % en poids d'initiateur de polymérisation,
rapporté chaque fois à la masse totale du matériau dentaire.

9. Matériau dentaire selon la revendication 8, **caractérisé en ce qu'**il comprend comme composant supplémentaire (d) 0 à 90 % en poids, préférentiellement 0 à 80 % en poids de matière de charge, rapporté à la masse totale du matériau dentaire.

10. Utilisation d'un xérogel chromophorique de la formule I :
(Si0₂)ₐ(Si0 _{1,5}-Sp-X-CG) (MeₙOₘ)b
dans laquelle :
Me est un métal de groupe principal du IIème au IIIème groupe principal ou un métal de transition du IVème groupe auxiliaire du système périodique des éléments ;
n est 1 ou 2, m étant égal à 1 ou 2, lorsque n est égal à 1 et m étant égal à 3, lorsque n est égal à 2 ;
Sp est un reste alkylène en C₁ à C₁₀ ou un reste oxyalkylène en C₂ à C₁₀ avec 1 à 4 atomes d'oxygène, ou est absent;
X est un groupe de liaison tel que CO-O, O-CO, CO-NH, NH-CO, O-CO-NH, NH-CO-O, NR, O, ou S, ou est absent, R étant hydrogène ou un reste alkyle en C₁ à C₆-alkyle ;
CG est un groupe 4-nitrophényle, un groupe azoïque aromatique, un groupe diarylméthane ou triarylméthane, un groupe xanthène ou un groupe anthrachinone ;
a est un nombre entier de 0 à 20, et
b est un nombre entier de 0 à 5, pour la fabrication d'un matériau dentaire.

11. Utilisation selon la revendication 10, pour la fabrication d'un matériau dentaire photodurcissable.

12. Utilisation selon la revendication 10 ou 11, pour la fabrication de ciments, matériaux de parement ou composites de plombage.
